## Europäisches Patentamt

(19) **European Patent Office**

**Office européen des brevets**

(11) Publication number: **0 174 978**
**B1**

---

(12) **EUROPEAN PATENT SPECIFICATION**

(45) Date of publication of patent specification: **07.11.90**

(51) Int. Cl.⁵: **A 61 K 37/02,** A 61 K 31/28,
A 61 K 31/40, A 61 K 31/195

(21) Application number: **85901679.2**

(22) Date of filing: **01.03.85**

(86) International application number:
**PCT/US85/00327**

(87) International publication number:
**WO 85/03871 12.09.85 Gazette 85/20**

---

(54) **METHOD OF PROPHYLAXIS OR TREATMENT OF ATHEROSCLEROSIS.**

---

(30) Priority: **01.03.84 US 585287**

(43) Date of publication of application:
**26.03.86 Bulletin 86/13**

(45) Publication of the grant of the patent:
**07.11.90 Bulletin 90/45**

(84) Designated Contracting States:
**DE FR GB**

(56) References cited:
**LU-A- 67 165**
**US-A-3 873 296**
**US-A-4 167 564**
**US-A-4 218 474**
**US-A-4 435 424**
**Chemcial Abstracts, Vol.98,1983,Kaliman,
Biogenic Monoamines and Their Precursors in
Rats with Spontaneous Arterial Hypertension,
abst. no. 195890u**
**Chemical Abstracts, Vol. 90, 1979,Shukla,
Species Variation in Manganese Induced
Changes in Brain Biogenic Amines, abst. no.
198507u**

(73) Proprietor: **ERK, Vernon
Cedar Hill Associates, Ste. 2860 420 Lexington
Ave.
New York, NY 10170 (US)**

(72) Inventor: **ERK, Vernon
Cedar Hill Associates, Ste. 2860 420 Lexington
Ave.
New York, NY 10170 (US)**

(74) Representative: **Perry, Robert Edward et al
GILL JENNINGS & EVERY 53-64 Chancery Lane
London WC2A 1HN (GB)**

(56) References cited:
**The Americal Journal of Clinical Nutrition, Vol.
6, No. 5, 1958,Salmon, The Significance of
Amino Acid Imbalance in Nutrition, pages
487-494**
**Nutrition Reviews, Vol. 32, No. 51974,
Metabolism of Alpha-keto Analogues of
Essential Amino Acids, pages 147-149**
**American Heart Journal, Vol. 82, no.3, 1971,
Rosenbloom, Progeria of Hutchenson-Gilford:A
caricature of aging, pages 287-289**

---

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## EP 0 174 978 B1

(56) References cited:
Proceedings of the Third International
Symposium Freising, Fed. Rep. of Germany,
July 1977, Frieden, Mode of Metal Metabolism
in Mammals, pages 8-14

Proceedings of the Third International
Symposium Freising, Fed. Rep. of Germany,
July 1977, Lassiter, Role of Bile and Diet on
54Mn Metabolism in Animals, pages 140-143

Proceedings of the Third International
Symposium Freising, Fed. Rep. of Germany,
July 1977, Pirchner, Genetical Aspects of Trace
Mineral Research, pages 646-649

## Description

This invention relates to compositions for use in the treatment of atherosclerosis and Hutchinson-Gilford's syndrome.

The two-carbon breakdown products of lipids and fats have been implicated in the synthesis of the lipids constituting lipoproteins and in finding their way into atheromatous plaques.

Catecholamines facilitate increased formation of acetyl CoA. Active isoprene units are formed from this "active acetate" and polymerise into the isoprenoids, including cholesterol. Diabetic and hypertensive patients tend to have an elevated level of catecholamines, and are especially susceptible to atheromatosis.

The two-carbon units are formed in relation to the balance of chemical activity in the tricarboxylic acid cycle in the mitochondrion. Maintaining the proper balance of carbohydrate and lipid breakdown in that organelle is central to avoiding excessive accumulation of lipids such as cholesterol.

Monoamine oxidase (MAO) has been found in all classes of vertebrates so far examined (1970): mammals, birds, reptiles, amphibians and teleosts. The enzyme occurs in many different tissues particularly in glands, plain muscle and the nervous system. In man, the parotid and submaxillary glands seems to be the richest source of MAO. It also occurs in molluscs and plants.

Inhibition of MAO leads to a very pronounced increase in the levels of norepinephrine in the sympathetic nervous system and of the monoamines serotonin, norepinephrine and dopamine in the monoamine-containing neurones of the CNS. Large amounts of amine now accumulate in the cytoplasm. The storage sites rapidly become filled to capacity with the transmitter. This enhanced accumulation of neuroamines within the neurones is presumed to be the basis for the anti-depressant action of the MAO inhibitors. It should be added that the presence in the urine of large amounts of unmetabolised serotonin and 3-O-methylated catecholamines is characteristic of patients on MAO inhibitor anti-depressants. These urinary compounds indicate clearance of the above amines from the blood and is consistent with an increased turnover rate of increased amounts of each amine.

In 1957, iproniazid was introduced for the treatment of depression. It has been studied extensively and is a MAO inhibitor. However, it has a variety of effects, besides the effect on depression. These have frequently posed problems. The use of these drugs has continued to be empirical. Iproniazid was removed from the market because of severe liver toxicity. It is interesting to note that such drugs exert their beneficial effect in depressed patients anywhere from one to several weeks after treatment is begun. In some instances, the improvement may progress to a state of euphoria, hypomania or even mania. Central stimulatory effects are seen with these drugs in normal individuals as well as in depressed patients. Other effects are orthostatic hypotension, allergic reactions affecting the liver, dizziness and a number of anti-cholinergic type symptoms.

A number of syndromes exhibit molar hypoplasia and micrognathia. Disturbances in bone growth generally are also likely to occur. For example, Cockayne's, Hallerman-Streiff's, Hutchinson-Gilford's, Seckel's and Treacher-Collins's syndromes may show some of these features. When the above are combined with atrophy of facial fatty tissue, the appearance of these dwarfs becomes striking. Of the various syndromes, the most interesting is Hutchinson-Gilford's. It is now usually referred to as progeria. Progeriacs usually die at about 10 to 15 years of age. A likely secondary cause of death is advanced hardening of the arteries (atherosclerosis). The cause of death primarily relates to that form of ageing associated with abnormal lipid deposits in the arteries. They appear to be classic cases of atheromatosis occurring at a very early age.

LU-A-0067165 discloses the use of manganese and tryptophan for the treatment of obesity. US-A-4167564 discloses a stabilised metal proteinate for enhancing the uptake of essential minerals in warm-blooded animals, which can include manganese and tryptophan.

Manganese-containing pharmaceutical preparations decelerate the rate of oxidation of biogenic amines. The resultant increased level of amines causes increased cellular activity. Shifts in biological parameters occur. By combining the use of manganese with tryptophan, the precursor of serotonin, the elevated ratio of catecholamines/serotonin can be restored to its original level.

Thus the invention relates to the use of a composition in the manufacture of a medicament for use in prophylaxis or treatment of Hutchinson-Gilford's syndrome or atherosclerosis in a warm-blooded animal, wherein the composition comprises (a) an amino-acid selected from L-tryptophan, D-tryptophan, an α-keto or α-hydroxy analogue thereof, acetyl-L-tryptophan and acetyl-D-tryptophan, or a dipeptide or tripeptide thereof, or a pharmaceutically-acceptable acid addition salt thereof, and (b) a manganese compound.

Manganese-containing pharmaceutical preparations are adapted, for use in the invention, for use in the system of oxidation of amines in biological systems. It is the overall concept of the present invention to control the rates of amine oxidation by providing these compounds.

In order that the invention may be more easily understood, the following evaluations are given, by way of illustration only, to show details of the formulation of the invention and the proposed clinical results obtaining using such formulations.

Standard methods of evaluation of atheromatosis and atherosclerosis are employed. Periodic laboratory determinations of lipid profiles and associated fundoscopic examinations are convenient. For the Hutchinson-Gilford's syndrome, the relative hypertension that develops and its response to pharmaceutical agents can be correlated to see if there is recession or diminished formation of lesions. For the adult hypertensive and/or diabetic, similar studies can be used to see if a correlation between the use of these agents and the long-term diminution of lesions or diminished formation of lesions can be demonstrated.

Evaluation of Hutchinson-Gilford's syndrome

Trial with tryptophan might well be conducted first.

Range of intake: tryptophan: 2/3 to 2 mg/kg body weight; manganese (calculated as manganese gluconate) 14 to 40 µg/kg body weight; combined treatment: begin with lowest dose of each, progress by steps.

Blood pressure (systolic, diastolic and pulse pressure) and lipoprotein profile should be correlated with each advance in dosage level.

The monitoring of blood pressure is the best means of monitoring the effects of manganese and tryptophan. It may be necessary to use other than the oral route. For parenteral use of manganese, there should be a clear knowledge of the status of the gastrointestinal tract.

The objective of the treatment should be to maintain the blood pressure within normal limits.

Evaluation of adult hypertension

Patients can readily be tested with lipid profiles and fundoscopic examinations. However, a much longer interval of time would be required than for the progeriacs. Because of the ease of monitoring the blood pressure, the effects of the manganese and the tryptophan can be determined in relation to laboratory values.

The range of intake will vary depending upon the stage of the disease. The individual clinician will need to determine this from visit to visit initially. 50 to 150 mg of tryptophan is a representative figure. The manganese may vary considerably initially. Values of 12.5 to 25 mg manganese gluconate may be encountered at first.

The objective of the treatment should be to maintain the blood pressure within normal limits. This provides a convenient method of evaluating episodes of stress in the life of the patient and the effect both upon blood pressure and of atheromatous changes and lipoprotein profile.

## Claims

1. Use of a composition for the manufacture of a medicament for use in prophylaxis or treatment of atherosclerosis in a warm-blooded animal, wherein the composition comprises (a) an amino-acid selected from L-tryptophan, D-tryptophan, an α-keto or α-hydroxy analogue thereof, acetyl-L-tryptophan and acetyl-D-tryptophan, or a dipeptide or tripeptide thereof, or a pharmaceutically-acceptable acid addition salt thereof, and (b) a manganese compound.

2. Use of a composition as defined in claim 1, for the manufacture of a medicament for use in treating Hutchinson-Gilford's syndrome.

## Patentansprüche

1. Verwendung einer Zusammensetzung für die Herstellung eines Arzneimittels zur Prophylaxe oder Behandlung von Atherosklerose bei Warmblütern, dadurch gekennzeichnet,m daß die Zusammensetzung umfaßt:
    a) eine Aminosäure ausgewählt aus L-Tryptophan, D-Tryptophan, einem α-Keto oder α-Hydroxy Analogon davon, Acetyl-L-Tryptophan und Acetyl-D-Tryptophan, oder ein Dipeptid oder Tripeptid davon, oder ein pharmazeutisch akzeptables Säureadditionssalz davon, und b) eine Manganverbindung.

2. Verwendung einer Zusammensetzung nach Anspruch 1 für die Herstellung eines Arzneimittels zur Behandlung des Hutchinson-Gilford Syndroms.

## Revendications

1. Utilisation d'une composition pour la préparation d'un médicament utilisable en prophylaxie ou dans le traitement de l'athérosclérose chez un animal à sang chaud, dans laquelle la composition comprend (a) un acide aminé choisi parmi le L-tryptophane, le D-tryptophane, un α-céto ou α-hydroxy analogue de ceux-ci, l'acétyl-L-tryptophane et l'acétyl-D-tryptophane, ou un dipeptide ou tripeptide de ceux-ci, ou un sel d'addition d'acide acceptable du point de vue pharmaceutique de ceux-ci, et (b) un composé du manganese.

2. Utilisation d'une composition selon la revendication 1, pour la préparation d'un médicament utilisable dans le traitement du syndrome de Hutchinson-Gilford.